# EUROPÄISCHE PATENTSCHRIFT

(11) **EP 2 185 125 B1**
(45) Veröffentlichungstag und Bekanntmachung des Hinweises auf die Patenterteilung: **21.02.2018**
(21) Anmeldenummer: 08801507.8
(22) Anmeldetag: 01.08.2008
(51) Int. Cl.: A61K 8/44, A61K 8/81, A61Q 17/04, A61K 8/06, A61Q 19/00

(54) **KOSMETISCHE EMULGATORKOMBINATION**
COSMETIC EMULSIFIER COMBINATION
COMBINAISON D'ÉMULSIFIANTS COSMÉTIQUES

(30) Priorität: 09.08.2007 DE 102007038413
(43) Veröffentlichungstag der Anmeldung: 19.05.2010
(73) Patentinhaber: Beiersdorf AG, 20253 Hamburg (DE)
(72) Erfinder: TESCH, Mirko, 22303 Hamburg (DE); SKUBSCH, Kerstin, 25497 Prisdorf (DE); BLOHM, Alexandra, 20257 Hamburg (DE)
(86) Internationale Anmeldenummer: PCT/EP2008/006367
(87) Internationale Veröffentlichungsnummer: WO 2009/018975

(56) Entgegenhaltungen:
- EP-A- 1 077 059
- EP-A- 1 958 609
- WO-A-03/097005
- US-A- 4 675 384
- US-A- 5 393 810
- US-A1- 2006 084 586
- US-A1- 2007 161 524
- ANONYM: RESEARCH DISCLOSURE, [Online] Bd. 508, Nr. 054, 10. August 2006 (2006-08-10), XP002509059 Emsworth, GB Gefunden im Internet: URL:http://www.rdjournal.co.uk/rd/search/R D508054.pdf> [gefunden am 2008-12-23]
- ANONYM: RESEARCH DISCLOSURE, [Online] Bd. 510, Nr. 067, 10. Oktober 2006 (2006-10-10), XP002509064 Emsworth, GB Gefunden im Internet: URL:http://www.rdjournal.co.uk/rd/search/R D510067.pdf> [gefunden am 2008-12-23]

## Beschreibung

Die vorliegende Erfindung betrifft eine kosmetische Emulsion.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement. Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 11 wasserlösliche, hydrophile. Emulgatoren mit einem besonders hohen HLB-Wert (größer 13) können auch als waschaktive Tenside eingesetzt werden.

Emulsionen des Standes der Technik haben den Nachteil, nur begrenzt Elektrolyt-stabil zu sein. Elektrolyte ändern im gelösten Zustand die Polarität der beiden Phasen, insbesondere die der wässrigen Phase, und machen eine Emulsion damit instabil (temperatur-, lager-, transportinstabil). Die Instabilität führt dabei zu Ölabscheidungen und Phasentrennungen. Durch den Einsatz von Elektrolyten in Emulsionen ändert sich darüber hinaus deren Viskosität.

Es war daher die Aufgabe der vorliegenden Erfindung ein Emulgatorsystem zu entwickeln, dass sich durch eine hohe Toleranz gegenüber dem Elektrolytgehalt und der Art der eingesetzten Elektrolyten auszeichnet. Die zu entwickelnde Emulsion sollte dabei unterschiedlich hohe (und auch sehr große) Mengen an Elektrolyt vertragen können, ohne instabil zu werden.

Darüber hinaus war es die Aufgabe der vorliegenden Erfindung, eine Emulsion zu entwickeln, deren Viskosität sich problemlos unabhängig von dem Elektrolytgehalt der Zubereitung über einen weiten Viskositätsbereich (von dünnflüssig bis cremartig zäh) einstellen lässt.

Es war eine weitere Aufgabe der vorliegenden Erfindung, ein besonders stabiles und sensorisch attraktives Emulgatorsystem zu entwickeln.

Nicht zuletzt war es die Aufgabe der vorliegenden Erfindung ein besonders einfaches und kostengünstig herstellbares Emulgatorsystem zu entwickeln.

Überraschend gelöst werden die Aufgaben durch eine kosmetische Zubereitung gemäß Anspruch 1.

Die erfindungsgemäßen Zubereitungen zeichnen sich durch ein besonders leichtes, wenig fettiges Hautgefühl aus. Darüber hinaus ziehen die erfindungsgemäßen Zubereitungen überraschend schnell in die Haut ein.

Zwar kennt der Fachmann die EP 0 766 959, doch konnte diese Schrift ebenso wenig den Weg zur vorliegenden Erfindung weisen, wie die US 2007/161524, WO 03/097005, EP 1077059, US 4675384, US 5393810, EP1958609, US 2006/00084586 sowie die Research disclosure Bd.508, Nr. 054 10. August 2006 (XP002509059) und Bd, 510, Nr. 067 10. Oktober 2006 (XP002509064).

Insbesondere war es für den Fachmann überraschend, dass bei dünnflüssigen erfindungsgemäßen Formulierungen mit einer Viskosität von z.B. 500 mPas die Zubereitungen trotz eines hohen Elektrolytgehaltes in der Wärme über längere Zeit stabil sind und sich sehr fein versprühen lassen.

Eine weitere überraschende und besonders vorteilhafte Eigenschaft dünnflüssiger erfindungsgemäßer Zubereitungen ist die Sprühbarkeit "über Kopf", d.h. der Verbraucher kann die Emulsion aus verschiedenen Positionen am ganzen Körper einfach applizieren. Hierfür gibt
es z.B. von der Firma Seaquist Sprühpumpen, welche diese Art der Applikation erlauben (PZ2 190DL USD1)

Nicht in den Schutzbereich der vorliegenden Erfindung fallen die folgenden Rezepturen:

| | **1** | **2** | **3** | **4** |
|---|---|---|---|---|
| Glyceryl Stearat | | | 2 | |
| Natrium Stearoylglutamat | 0,05 | 0,1 | 0,2 | 0,5 |
| Stearylalkohol | | | 2 | |
| Copolymer aus Vinylpyrrolidon und Acrylsäure | 2 | 1,6 | 0,5 | 1 |
| Carbomer | | 0,2 | | |
| C₁₂₋₁₅ Alkyl Benzoat | | 5 | 1 | 10 |
| Butylenglycol Dicaprylat/Dicaprat | 2 | 8 | | 10 |
| Phenethylbenzoat | | | 2 | 5 |
| Octyldodekanol | | 2 | 4 | |
| Myristyl Myristat | | | | 1 |
| Caprylic/Capric Triglycerid | | | 2 | |
| Isodecyl Neopentanoate | 3 | | | |
| Cyclomethicon | 2 | | | |
| Dimethicon | 2 | | | |
| Glycerin | 10 | 2 | 5 | 8 |
| Bis-Ethylhexyloxyphenol Methoxyphenyl Triazin | | 1 | | 2 |
| Butyl Methoxydibenzoylmethan | | 4 | | 3 |
| Polysilicon-15 | | | 2 | |
| Ethylhexyl Methoxycinnamat | | 3 | | |
| Octylsalicylat | | 3 | 2 | |
| Phenylbenzimidazol Sulfonsäure | 1 | 0,5 | 1 | 2,5 |
| Phenylen-1,4-bis-(2-benzimidazyl)-3,3',5,5'-tetrasulfonsäure | 1 | | 3 | 0,5 |
| Ethylhexyltriazin | 2 | | | 3 |
| Titandioxid | 2 | | | |
| Aluminium Stärke Octenylsuccinat | 2 | | | 2 |
| Natrium Stärke Octenylsuccinat | | | 3 | |
| Tapioka Stärke | 2 | | | |
| Alkohol | | | 4 | |
| Folsäure | | 0,03 | 0,02 | |
| Q 10 | 0,05 | | | |
| Na₂H₂EDTA | 0,2 | 0,2 | 0,2 | 0,5 |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Wasser | ad 100,0 | ad 100,0 | ad 100,0 | ad 100,0 |

Es ist erfindungsgemäß vorteilhaft, wenn die erfindungsgemäße Zubereitung in Form einer O/W-Emulsion vorliegt.

Es ist erfindungsgemäß vorteihaft, wenn als vernetztes Polymer aus Vinylpyrrolidon und Acrylsäure die Verbindung mit der CAS No : 527685-31-0 eingesetzt wird.

Vernetztes Polymer aus Vinylpyrrolidon wird dabei erfindungsgemäß vorteilhaft in einer Menge von 0,05 bis 4 Gewichts-% und bevorzugt in einer Menge von 0,1 bis 3 Gewichts-%, bezogen auf das Gesamtgewicht der Zubereitung eingesetzt.

Erfindungsgemäß vorteilhaft enthält die erfindungsgemäße Zubereitung Natriumstearoylglutamat in einer Konzentration von 0,05 bis 2 Gewichts-% und erfindungsgemäß bevorzugt in einer Konzentration von 0,1 bis 1 Gewichts-%, jeweils bezogen auf das Gesamtgewicht der Zubereitung.
Das erfindungsgemäße Natriumstearoylglutamat kann beispielsweise unter dem Namen Eumulgin SG bei der Firma Cognis erworben werden.
Es ist erfindungsgemäß vorteilhaft, wenn die erfindungsgemäße Zubereitung Sucrosepolystearat enthält.
Sucrosepolystearat wird dabei erfindungsgemäß vorteilhaft in einer Menge von 0,1 bis 6 Gewichts-% und bevorzugt in einer Menge von 0,2 bis 4 Gewichts-%, bezogen auf das Gesamtgewicht der Zubereitung eingesetzt.
Erfindungsgemäß vorteilhafte Ausführungsformen der vorliegenden Erfindung sind dadurch gekennzeichnet, dass die Zubereitung hydriertes Polyisobuten enthält.
Hydriertes Polyisobuten wird dabei erfindungsgemäß vorteilhaft in einer Menge von 0,05 bis 2 Gewichts-% und bevorzugt in einer Menge von 0,1 bis 1 Gewichts-%, bezogen auf das Gesamtgewicht der Zubereitung eingesetzt.
Es ist erfindungsgemäß vorteilhaft, wenn die Zubereitung Elektrolyte enthält.
Zu den Elektrolyten im Sinne der vorliegenden Erfindung zählen organische und anorganische Salze.
Es ist dabei erfindungsgemäß vorteilhaft, wenn die Salze als Kationen Natrium-, Kalium-Triethanolammonium- Ammonium-, Calcium- Magnesiumionen enthalten.
Als Anionen der Salze können beispielsweise Chloride, Phosphate, Carbonate, Hydrogencarbonate, Sulfate, Hydrogensulfate, Citrate, Lactate, EDTA, Salicylate, Ascorbate, Acetate, Benzoate und weitere Carbonsäureanionen eingesetzt werden.

Es ist dabei erfindungsgemäß vorteilhaft, wenn der Elektrolytgehalt von 0,05 bis 10 Gewichts-% und bevorzugt von 0,1 bis 5 Gewichts-% beträgt, bezogen auf das Gesamtgewicht der Zubereitung.
Es ist erfindungsgemäß bevorzugt, wenn die Zubereitung einen oder mehrere UV-Filter gewählt aus der Gruppe Phenylen-1,4-bis-(2-benzimidazyl)-3,3'-5,5'-tetrasulfonsäuresalze; 2-Phenylbenzimidazol-5-sulfonsäuresalze; 4-(2-Oxo-3-bornylidenmethyl)benzolsulfonsäuresalze; 2-Methyl-5-(2-oxo-3-bornylidenmethyl)sulfonsäuresalze; Terephthalidendicamphersulfonsäuresalze enthält.
Phenylen-1,4-bis-(2-benzimidazyl)-3,3'-5,5'-tetrasulfonsäuresalze werden dabei erfindungsgemäß vorteilhaft in einer Menge von 0,1 bis 10 Gewichts-% und bevorzugt in einer Menge von 0,5 bis 5 Gewichts-%, bezogen auf das Gesamtgewicht der Zubereitung eingesetzt.
Phenylbenzimidazol-5-sulfonsäuresalze werden dabei erfindungsgemäß vorteilhaft in einer Menge von 0,1 bis 8 Gewichts-% und bevorzugt in einer Menge von 0,5 bis 5 Gewichts-%, bezogen auf das Gesamtgewicht der Zubereitung eingesetzt.
Terephthalidendicamphersulfonsäuresalze werden dabei erfindungsgemäß vorteilhaft in einer Menge von 0,1 bis 10 Gewichts-% und bevorzugt in einer Menge von 0,5 bis 5 Gewichts-%, bezogen auf das Gesamtgewicht der Zubereitung eingesetzt.
Erfindungsgemäß vorteilhafte Ausführungsformen der vorliegenden Erfindung sind dadurch gekennzeichnet, dass die Zubereitung UV-Filter enthält, gewählt aus der Gruppe der Verbindungen 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1,-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 4-(tert.-Butyl)-4'-methoxydibenzoylmethan; 4-Dicyanomethylen-2,6-dimethyl-1,4-dihydropyridin-N-(ethyloxysulfatestersalze), Titandioxid; Zinkoxid; Polysiloxancopolymer mit einer statistischen Verteilung gemäß der Formel:

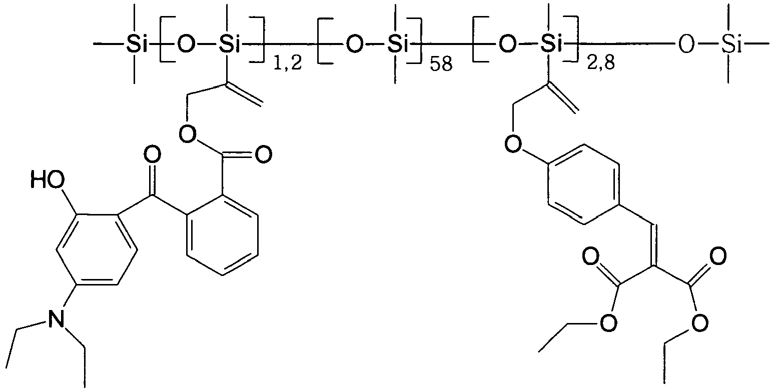

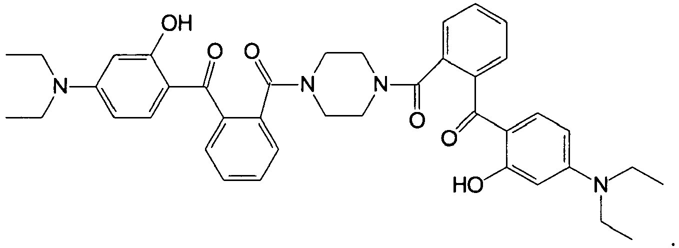

Es ist erfindungsgemäß vorteilhaft, wenn die erfindungsgemäße Zubereitung frei ist von p-Methylbenzylidencampher.

Es ist erfindungsgemäß vorteilhaft, wenn die Zubereitung Stärke und/oder deren Derivate enthält. Dabei ist der Einsatz von Tapioka erfindungsgemäß bevorzugt.

Stärke und/oder deren Derivate wird dabei erfindungsgemäß vorteilhaft in einer Menge von 0,05 bis 8 Gewichts-% und bevorzugt in einer Menge von 0,1 bis 5 Gewichts-%, bezogen auf das Gesamtgewicht der Zubereitung eingesetzt.

Es ist erfindungsgemäß vorteilhaft, wenn die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate und/oder Licochalcon A enthält.

Derartige Wirkstoffe können in einer Einzelkonzentration von 0,01 bis 10 Gewichts-%, bezogen auf das Gesamtgewicht der Zubereitung vorteilhaft eingesetzt werden.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Phytin, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.
Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.
Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.
Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.
Es ist erfindungsgemäß vorteilhaft, wenn die Zubereitung ein oder mehrere Parfümstoffe gewählt aus der Gruppe der Verbindungen Limonen [5989-27-5], Citral, Linalool [78-70-6], alpha-Isomethylionon [1335-46-2], Geraniol [106-24-1], Citronellol [106-22-9], [24851-98-7], [18479-58-8], [54464-57-2], [80-54-6], [1222-05,5], [32388-55-9], [105-95-3], [31906-04-4], [8008-57-9], [32210-23-4], [120-57-0], [115-95-7], [101-86-0], [140-11-4], [6259-76-3] und [127-51-5], 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethyl-propionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropional, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linalylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Es ist dabei erfindungsgemäß vorteilhaft, wenn die erfindungsgemäße Zubereitung ein oder mehrere Parfümstoffe in einer Gesamtkonzentration von 0,001 bis 1 Gewichts-%, bezogen auf das Gesamtgewicht der Zubereitung, enthält.

Es ist dabei erfindungsgemäß bevorzugt, wenn die erfindungsgemäße Zubereitung ein oder mehrere Parfümstoffe in einer Gesamtkonzentration von 0,001 bis 0,5 Gewichts-%, bezogen auf das Gesamtgewicht der Zubereitung, enthält.

Erfindungsgemäß vorteilhaft enthält die erfindungsgemäße Zubereitung eine oder mehrere Verbindungen gewählt aus der Gruppe der Verbindungen Isopropanol, Propylenglykol, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, 2-Methyl-1,3-propandiol; 1,2-Pentandiol; 1,2-Hexandiol, 1,2-Octandiol.

Es ist erfindungsgemäß bevorzugt, wenn die Zubereitung ein oder mehrere Diole gewählt aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol enthält.

Derartige Diole können erfindungsgemäß vorteilhaft in einer Konzentration von 0,1 bis 20 Gewichts-%, bezogen auf das Gesamtgewicht der Zubereitung, in dieser enthalten sein.

Es ist erfindungsgemäß vorteilhaft, wenn die Zubereitung ein oder mehrere Parabene (z.B. Methyl-, Ethyl-, Propyl-, Butylparaben) enthält.

Dabei ist ein Gesamt-Paraben-Gehalt von 0,05 bis 1 Gewichts-%, bezogen auf das Gesamtgewicht der Zubereitung erfindungsgemäß vorteilhaft.

Erfindungsgemäß bevorzugt ist die erfindungsgemäße Zubereitung frei von Polyethylenglycolen, d.h. PEG-frei.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 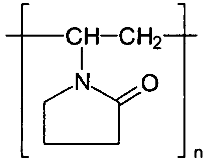 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen.

Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.
Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.
Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Elektrolyte, organische Lösungsmittel oder Silikonderivate.
Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.
Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.
Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.
Erfindungsgemäß ist die Verwendung der Zubereitung in kosmetischen Sprays (insbesondere Sonnenschutzmitte n).
Erfindungsgemäß ist die Verwendung für die Tränkung kosmetischer Tücher.

### Vergleichsversuche

| | **A** | **B** | **C** | **D** |
|---|---|---|---|---|
| **Rohstoff** | **m [%]** | **m [%]** | **m [%]** | **m [%]** |
| Sodium Stearoyl Glutamat | 0,2 | 0,2 | 0,2 | 0,2 |
| Copolymer aus Vinylpyrrolidon und Acrylsäure | 0,6 | 0,8 | 1,0 | 1,2 |
| Alkohol | 2,0 | 2,0 | 2,0 | 2,0 |
| Bis-Ethylhexyloxyphenol Methoxyphenyl Triazin | 1,0 | 1,0 | 1,0 | 1,0 |
| Butyl Methoxydibenzoylmethan | 4,5 | 4,5 | 4,5 | 4,5 |
| Polysilicon-15 | 2,0 | 2,0 | 2,0 | 2,0 |
| Cyclomethicon | 4,0 | 4,0 | 4,0 | 4,0 |
| C₁₂₋₁₅ Alkyl Benzoat | 4,0 | 4,0 | 4,0 | 4,0 |
| Parfüm | 0,4 | 0,4 | 0,4 | 0,4 |
| Glycerin | 4,0 | 4,0 | 4,0 | 4,0 |
| Octocrylen | 9,0 | 9,0 | 9,0 | 9,0 |
| Phenylbenzimidazole Sulfonsäure | 3,0 | 3,0 | 3,0 | 3,0 |
| Sucrose Polystearat + hydriertes Polyisobuten | 1,0 | 1,0 | 1,0 | 1,0 |
| Trisodium EDTA | 1,0 | 1,0 | 1,0 | 1,0 |
| VP/Hexadecene Copolymer | 0,5 | 0,5 | 0,5 | 0,5 |
| Konservierungsmittel | 0,5 | 0,5 | 0,5 | 0,5 |
| Water | ad 100 | ad 100 | ad 100 | ad 100 |
| **Viskosität mPas bei 25°C (Haake VT02** | 500 | 1500 | 3000 | 4300 |
| **Stabilität nach 3 Monaten 40°C Lagerung** | sehr gut | sehr gut | sehr gut | sehr gut |

Die Emulsion A ist außerdem überkopf-sprühbar mithilfe z.B. der Seaquist-Pumpe PZ2 190DL USD1.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Emulsion** | **1** | **2** | **3** | **4** | **5** | **6** |
|---|---|---|---|---|---|---|
| Natrium Stearoylglutamat | 0,2 | 0,1 | 0,2 | 0,4 | 0,1 | 0,5 |
| Sucrosepolystearat + hydriertes Polyisobuten | 1 | | 1 | 0,6 | 0,5 | |
| Copolymer aus Vinylpyrrolidon und Acrylsäure | 0,2 | 0,3 | 0,4 | 0,7 | 1 | 1,5 |
| Acrylat/C10-30 Alkyl Acrylat Cross polymer | 0,3 | 0,2 | | | 0,2 | |
| C₁₂₋₁₅ Alkyl Benzoat | 5 | | | | 8 | 3 |
| Butylenglycol Dicaprylat/Dicaprat | 2 | | 7 | | | 3 |
| Triheptanoin | 3 | | | | | |
| Octyldodekanol | | | 3 | | | |
| Dicaprylylcarbonat | 3 | 3 | | | | |
| Caprylic/Capric Triglycerid | | | | | 2 | |
| Isodecyl Neopentanoat | | 2 | | | | |
| Cyclomethicon | | | 5 | 3 | | 2 |
| Dimethicon | | | | 2 | | |
| Glycerin | 5 | 2 | 5 | 7 | 1 | 5 |
| Bis-Ethylhexyloxyphenol Methoxyphenyl Triazin | 2 | 0,5 | 2 | 1 | | |
| Ethylhexyltriazin | 2,5 | 1 | | | 2 | |
| Butyl Methoxydibenzoylmethan | 4 | 0,5 | 5 | 3 | 2 | |
| Diethylamino Hydroxybenzoyl Hexyl Benzoat | | 3 | | | | 5 |
| Polysilicon-15 | | | 3 | 2 | | |
| Ethylhexyl Methoxycinnamat | 8 | 5 | 5 | | | |
| Octylsalicylat | | | 5 | | | 5 |
| Homosalat | | | | | 3 | |
| Octocrylen | | 3 | | 10 | 5 | |
| Phenylbenzimidazol Sulfonsäure | | | 2 | 4 | | 3 |
| Titandioxid | | | | | 2 | |
| Folsäure | 0,05 | | 0,1 | | 0,2 | |
| Ethanol | 2 | | 4 | | 3 | 2,5 |
| Parfüm, Konservierunsmittel | q.s. | q.s. | q.s. | q.s. | q.s. | q.s. |
| Farbstoffe, Neutralisationsmittel etc | q.s. | q.s. | q.s. | q.s. | q.s. | q.s. |
| Wasser | ad 100,0 | ad 100,0 | ad 100,0 | ad 100,0 | ad 100,0 | ad 100,0 |

| **Beispiel** | **7** | **8** | **9** | **10** | **11** | **12** |
|---|---|---|---|---|---|---|
| Natrium Stearoylglutamat | 0,5 | 0,8 | 0,2 | 0,4 | 0,3 | 0,3 |
| Sucrose Polystearat + hydriertes Polyisobuten | | | 1 | 0,8 | 0,5 | |
| Copolymer aus Vinylpyrrolidon und Acrylsäure | 1,5 | 0,8 | 1 | 0,5 | 0,5 | 0,3 |
| Stearylalkohol | | | | | 0,5 | |
| Cetylalkohol | | 2 | | | | 2 |
| Acrylates/C₁₀₋₃₀ Alkyl Acrylat Crosspolymer | | | | 0,3 | 0,2 | 0,1 |
| Myristyl Myristat | | | 1 | | | |
| Tapioka Stärke | 2 | | 4 | | 1 | |
| C₁₂₋₁₅ Alkyl Benzoat | | 3 | 5 | | | |
| Butylenglycol Dicaprylat/Dicaprat | 2 | 4 | | | | |
| Dicaprylyl Carbonat | 4 | | 2 | | 2 | |
| Octyldodecanol | | | | | | 5 |
| Cocoglycerid | | 3 | | | | |
| Cyclomethicon | 3 | | | | 2 | |
| 2-Propylheptyloctanoat | 4 | 8 | 10 | 5 | 4 | 6 |
| Glycerin | 5 | 6 | 4 | 10 | 2 | 4 |
| Ethanol | 2 | | 1 | | | 4 |
| Butyl Methoxydibenzoylmethan | | 2 | 2 | | 3 | 4 |
| Ethylhexylmethoxycinnamat | 5 | | | | | |
| Octylsalicylat | | | | | 5 | |
| Octocrylen | 5 | | | | | |
| Dinatrium Phenylen-1,4-bis-(2-benzimidazyl)-3,3'-5,5'-tetrasulfonsäuresalz | 2 | | | 1,5 | | 2 |
| Phenylbenzimidazol Sulfonsäure | | 2 | 2 | | | 1 |
| Titandioxid | 1 | | | | | 0,5 |
| Dimethicondiethylbenzalmalonat (INCI: Polysilicon-15) | | 1 | 4 | | 0,5 | 3 |
| Ethylhexyltriazin | | | | 3 | | |
| Vitamin E Acetat | 0,5 | 0,2 | 0,1 | 0,5 | 0,25 | 0,3 |
| Na₂H₂EDTA | 0,1 | 0,2 | 0,2 | 0,2 | 0,2 | 0,5 |
| Parfüm | | | 0,2 | 0,1 | 0,3 | 0,25 |
| Konservierungsmittel | q.s. | q.s. | q.s. | q.s. | q.s. | q.s. |
| Natriumhydroxid, Farbstoffe | q.s. | q.s. | q.s. | q.s. | q.s. | q.s. |
| Wasser | ad 100,0 | ad 100,0 | ad 100,0 | ad 100,0 | ad 100,0 | ad 100,0 |

| **Emulsion** | **13** | **14** | **15** | **16** |
|---|---|---|---|---|
| Natrium Stearoylglutamat | 0,5 | 0,2 | 0,25 | 0,4 |
| Glyceryl Stearat Citrat | | 1 | 0,5 | |
| Cetearylalkohol | 2 | | | |
| Stearylalkohol | | | 2 | |
| Cetylalkohol | | | | 2,5 |
| Xanthan Gummi | | | 0,2 | |
| Copolymer aus Vinylpyrrolidon und Acrylsäure | 0,5 | 0,2 | 0,1 | 0,7 |
| Acrylates/C10-30 Alkyl Acrylate Cross polymer | | 0,2 | | |
| Carbomer | | | | 0,3 |
| 2-Propylheptyloctanoat | 5 | 3 | 7 | 10 |
| C₁₂₋₁₅ Alkyl Benzoat | 2 | | | |
| Cocoglyceride | | 3 | | |
| Isopropylpalmitat | | | 4 | |
| Jojobaöl | | 2 | | 5 |
| Caprylic/Capric Triglycerid | 2 | | | |
| Mandelöl | | | 3 | |
| 2-Methyl-1,3-propandiol | 4 | 2 | | |
| Glycerin | 2 | 5 | 10 | 5 |
| Shea Butter | | 1 | | 2 |
| Natrium Stärke Octenylsuccinat | 3 | | | |
| Tapioka Stärke | 1 | 2 | | |
| Alkohol | 1 | | 2 | |
| Kreatin | 0,5 | | | |
| Coenzym Q10 | | 0,1 | 0,2 | |
| Natriumascorbylphosphat | 0,5 | | | |
| Na₂H₂EDTA | 0,1 | 0,1 | 0,2 | |
| Methylparaben | 0,1 | 0,2 | 0,2 | 0,15 |
| Phenoxyethanol | 0,5 | 0,4 | 0,6 | 0,3 |
| Parfüm | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Wasser | ad 100,0 | ad 100,0 | ad 100,0 | ad 100,0 |

## Patentansprüche

1. Kosmetische Zubereitung in Form einer Hautschutzcreme, Tages- oder Nachtcreme, zum kosmetischen Lichtschutz oder Schminkprodukt in der dekorativen Kosmetik enthaltend
a) Natriumstearoylglutamat,
b) ein vernetztes Polymer aus Vinylpyrrolidon und Acrylsäure, ausgenommen die folgenden Rezepturen:
| | **1** | **2** | **3** | **4** |
|---|---|---|---|---|
| Glyceryl Stearat | | | 2 | |
| Natrium Stearoylglutamat | 0,05 | 0,1 | 0,2 | 0,5 |
| Stearylalkohol | | | 2 | |
| Copolymer aus vinylpyrrolidon und Acrylsäure | 2 | 1,6 | 0,5 | 1 |
| Carbomer | | 0,2 | | |
| C₁₂₋₁₅ Alkyl Benzoat | | 5 | 1 | 10 |
| Butylenglycol Dicaprylat/Dicaprat | 2 | 8 | | 10 |
| Phenethylbenzoat | | | 2 | 5 |
| Octyldodekanol | | 2 | 4 | |
| Myristyl Myristat | | | | 1 |
| Carylic/Capric Triglycerid | | | 2 | |
| Isodecyl Neopentanoate | 3 | | | |
| Cyclomethicon | 2 | | | |
| Dimethicon | 2 | | | |
| Glycerin | 10 | 2 | 5 | 8 |
| Bis-Ethylhexyloxyphenol Methoxyphenyl Triazin | | 1 | | 2 |
| Butyl Methoxydibenzoylmethan | | 4 | | 3 |
| Polysilicon-15 | | | 2 | |
| Ethylhexyl Methoxycinnamat | | 3 | | |
| Octylsalicylat | | 3 | 2 | |
| Phenylbenzimidazol Sulfonsäure | 1 | 0,5 | 1 | 2,5 |
| Phenylen-1,4-bis-(2-benzimidazyl)--3,3',5,5'-tetrasulfonsäure | 1 | | 3 | 0,5 |
| Ethylhexyltriazin | 2 | | | 3 |
| Titandioxid | 2 | | | |
| Aluminium Stärke Octenylsuccinat | 2 | | | 2 |
| Natrium Stärke Octenylsuccinat | | | 3 | |
| Tapioka Stärke | 2 | | | |
| Alkohol | | | 4 | |
| Folsäure | | 0,03 | 0,02 | |
| Q 10 | 0,05 | | | |
| Na₂H₂EDTA | 0,2 | 0,2 | 0,2 | 0,5 |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Wasser | ad 100,0 | ad 100,0 | ad 100,0 | ad 100,0 |

2. Kosmetische Zubereitung nach Anspruch 1, **dadurch gekennzeichnet, dass** als vernetztes Polymer aus Vinylpyrrolidon und Acrylsäure die Verbindung mit der CAS No : 527685-31-0 eingesetzt wird.

3. Kosmetische Zubereitung nach einem der vorhergehenden Ansprüche, **dadurch gekennzeichnet, dass** die Zubereitung Sucrosepolystearat enthält.

4. Kosmetische Zubereitung nach einem der vorhergehenden Ansprüche, **dadurch gekennzeichnet, dass** die Zubereitung hydriertes Polyisobuten enthält.

5. Kosmetische Zubereitung nach einem der vorhergehenden Ansprüche, **dadurch gekennzeichnet, dass** die Zubereitung Elektrolyte enthält.

6. Kosmetische Zubereitung nach einem der vorhergehenden Ansprüche, **dadurch gekennzeichnet, dass** die Zubereitung einen oder mehrere UV-Filter gewählt aus der Gruppe Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethy1)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; Tersphthalidendicamphersulfonsäuresalze enthält.

7. Kosmetische Zubereitung nach einem der vorhergehenden Ansprüche, **dadurch gekennzeichnet, dass** die Zubereitung UV-Filter enthält, gewählt aus der Gruppe der Verbindungen 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-di-phenylacrylat; Dimethicodiethylbenzalmalonat; 2-Ethylhexyl-2-cyano-3,3-diphenyl-acrylat; 3-(4-(2,2-bis Ethoxycarbonylvinyl)-phenoxy)propenyl)-methoxysiloxan/ Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 4-(tert.-Butyl)-4'-Methoxydibenzoylmethan; 4-Dicyanomethylen-2,6-dimethyl-1,4-dihydropyridin-N-(ethyloxysulfatestersalze), 2,4,6-Tris-(biphenyl)-1,3,5-triazin, 2,2'-Methylen-bis-(6-(2H-benzotriazol-2-yl)-4-(1,1,3,3-tetramethylbutyl)-phenol), Titandioxid; Zinkoxid; Polysiloxancopolymer mit einer statistischen Verteilung gemäß der Formel:

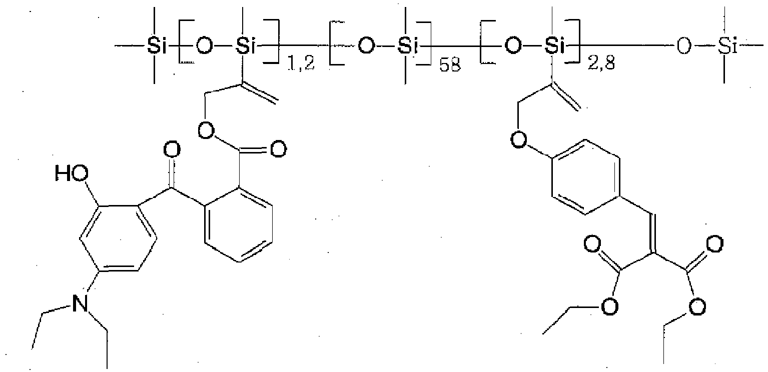

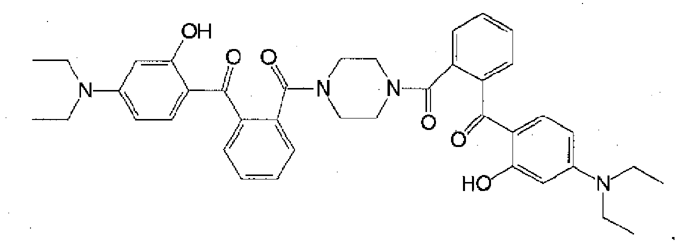

8. Kosmetische Zubereitung nach einem der vorhergehenden Ansprüche, **dadurch gekennzeichnet, dass** die Zubereitung Stärke und/oder deren Derivate enthält.

9. Kosmetische Zubereitung nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate und/oder Licochalcon A enthält.

10. Zubereitung nach einem der vorhergehenden Ansprüche, **dadurch gekennzeichnet, dass** die Zubereitung ein oder mehrere Parfümstoffe gewählt aus der Gruppe der Verbindungen Limonen [5989-27-5], Citral, Linalool [78-70-6], alpha-Isomethylionon [1335-46-2], Geraniol [106-24-1], Citronellol [106-22-9], [24851-98-7], [18479-58-8], [54464-57-2], [80-54-6], [1222-05,5], [32388-55-9], [105-95-3], [31906-04-4], [8008-57-9], [32210-23-4], [120-57-0], [115-95-7], [101-86-0], [140-11-4], [6259-76-3] und [127-51-5], 2-isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethyl-propionalcinnamal, Amylsalicylat, Amylcinnamylalkohol, Anisalköhol, Benzoin, Benzylalkohol, Benzylbenzoat, Benzylcinnamat, Benzylsalicylat, Bergamotöl, bitteres Orangenöl, Butylphenylmethylpropional, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linalylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

11. Kosmetische Zubereitung nach einem der vorhergehenden Ansprüche, **dadurch gekennzeichnet, dass** die Zubereitung ein oder mehrere Diole gewählt aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol, Ethylhexylglycerin enthält.

12. Kosmetische Zubereitung nach einem der vorhergehenden Ansprüche, **dadurch gekennzeichnet, dass** die Zubereitung ein oder mehrere Parabene (z.B. Methyl-, Ethyl-, Propyl-, Butylparaben) enthält.

## Claims

1. Cosmetic preparation in the form of a skin protection cream, day or night cream, for cosmetic protection from light, or make-up product in decorative cosmetics, comprising
a) sodium stearoyl glutamate,
b) a crosslinked polymer of vinylpyrrolidone and acrylic acid, with the exception of the following formulations:
| | **1** | **2** | **3** | **4** |
|---|---|---|---|---|
| Glyceryl Stearate | | | 2 | |
| Sodium Stearoylglutamate | 0.05 | 0.1 | 0.2 | 0.5 |
| Stearyl alcohol | | | 2 | |
| Copolymer of Vinylpyrrolidone and Acrylic acid | 2 | 1.6 | 0.5 | 1 |
| Carbomer | | 0.2 | | |
| C₁₂₋₁₅Alkyl Benzoate | | 5 | 1 | 10 |
| Butylene glycol Dicaprylate/Dicaprate | 2 | 8 | | 10 |
| Phenethyle benzoate | | | 2 | 5 |
| Octyldodecanol | | 2 | 4 | |
| Myrsityl Myristate | | | | 1 |
| Caprylic/Capric Triglyceride | | | 2 | |
| Isodecyl Neopentanoate | 3 | | | |
| Cyclomethicone | 2 | | | |
| Dimethicone | 2 | | | |
| Glycerol | 10 | 2 | 5 | 8 |
| Bis-Ethylhexyloxyphenol Methoxyphenyl Triazine | | 1 | | 2 |
| Butyl Methoxydibenzolmethane | | 4 | | 3 |
| Polysilicone-15 | | | 2 | |
| Ethylhexyl Methoxycinnamate | | 3 | | |
| Octyl salicylate | | 3 | 2 | |
| Phenylbenzimidazole Sulfonic acid | 1 | 0.5 | 1 | 2.5 |
| Phenylene-1,4-bis-(2-benzimidazyl)-3,3',5,5'-tetrasulfonic acid | 1 | | 3 | 0.5 |
| Ethylhexyltriazine | 2 | | | 3 |
| Titanium dioxide | 2 | | | |
| Aluminium Starch Octenylsuccinate | 2 | | | 2 |
| Sodium Starch Octenylsuccinate | | | 3 | |
| Tapioca Starch | 2 | | | |
| Alcohol | | | 4 | |
| Folic acid | | 0.03 | 0.02 | |
| Q10 | 0.05 | | | |
| Na₂H₂EDTA | 0.2 | 0.2 | 0.2 | 0.5 |
| Fragrance, Preservative | q.s. | q.s. | q.s. | q.s. |
| Dyes, etc. | q.s. | q.s. | q.s. | q.s. |
| Sodium hydroxide | q.s. | q.s. | q.s. | q.s. |
| Water | ad 100.0 | ad 100.0 | ad 100.0 | ad 100.0 |

2. Cosmetic preparation according to Claim 1, **characterized in that** the crosslinked polymer of vinylpyrrolidone and acrylic acid used is the compound with the CAS No: 527685-31-0.

3. Cosmetic preparation according to any one of the preceding claims, **characterized in that** the preparation comprises sucrose polystearate.

4. Cosmetic preparation according to any one of the preceding claims, **characterized in that** the preparation comprises hydrogenated polyisobutene.

5. Cosmetic preparation according to any one of the preceding claims, **characterized in that** the preparation comprises electrolytes.

6. Cosmetic preparation according to any one of the preceding claims, **characterized in that** the preparation comprises one or more UV filters selected from the group consisting of phenylene-1,4-bis(2-benzimidazyl)-3,3',5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; terephthalidenedicamphorsulfonic acid salts.

7. Cosmetic preparation according to any one of the preceding claims, **characterized in that** the preparation comprises UV filters selected from the group of the compounds 2-(2H-benzotriazol-2-yl)-4-methyl-6-[2-methyl-3-[1,3,3,3-tetramethyl-1-(trimethylsilyl)oxy]disiloxanyl)propyl]phenol; 3-(4-methylbenzylidene)camphor; 3-benzylidenecamphor; ethylhexyl salicylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethyl benzalmalonate; 2-ethylhexyl 2-cyano-3,3-diphenylacrylate; 3-(4-(2,2-bis-ethoxycarbonylvinyl)phenoxy)propenyl) methoxysiloxane/dimethylsiloxane copolymer; dioctylbutylamidotriazone (INCI: Diethylhexyl Butamidotriazone); 2,4-bis[5-1(dimethylpropyl)benzoxazol-2-yl(4-phenyl)imino]-6-(2-ethylhexyl)imino-1,3,5-triazine (CAS No. 288254-16-0); tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine; 4-(tert-butyl)-4'-methoxydibenzoylmethane; 4-dicyanomethylene-2,6-dimethyl-1,4-dihydropyridine N-(ethyloxysulfate ester salts), 2,4,6-tris(biphenyl)-1,3,5-triazine, 2,2'-methylenebis(6-(2H-benzotriazol-2-yl)-4-(1,1,3,3-tetramethylbutyl)phenol), titanium dioxide; zinc oxide; polysiloxane copolymer with a random distribution according to the formula:

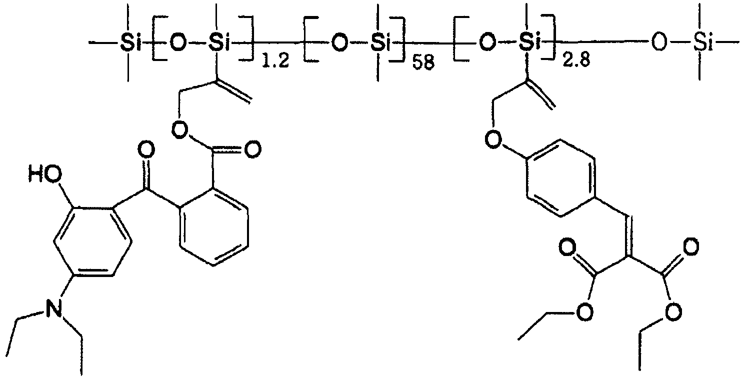

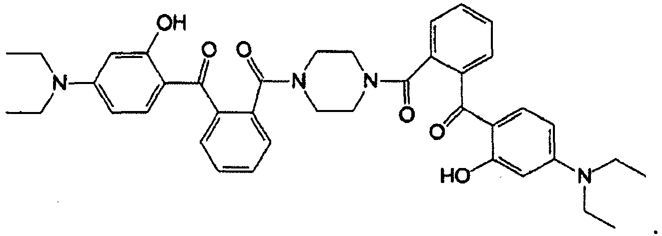

8. Cosmetic preparation according to any one of the preceding claims, **characterized in that** the preparation comprises starch and/or derivatives thereof.

9. Cosmetic preparation according to any one of the preceding claims, **characterized in that** the preparation comprises one or more compounds selected from the group of the compounds alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, tocopheryl acetate, dihydroxyacetone; 8-hexadecene-1,16-dicarboxylic acid, glycerylglycose, (2-hydroxyethyl)urea, vitamin E and its derivatives and/or licochalcone A.

10. Preparation according to any one of the preceding claims, **characterized in that** the preparation comprises one or more perfume substances selected from the group of the compounds limonene [5989-27-5], citral, linalool [78-70-6], alpha-isomethylionone [1335-46-2], geraniol [106-24-1], citronellol [106-22-9], [24851-98-7], [18479-58-8], [54464-57-2], [80-54-6], [1222-05-5], [32388-55-9], [105-95-3], [31906-04-4], [8008-57-9], [32210-23-4], [120-57-0], [115-95-7], [101-86-0], [140-11-4], [6259-76-3] and [127-51-5], 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic acid diester, alpha-amylcinnamaldehyde, alpha-methylionone, amyl C, butylphenylmethylpropional cinnamal, amyl salicylate, amyl cinnamyl alcohol, anise alcohol, benzoin, benzyl alcohol, benzyl benzoate, benzyl cinnamate, benzyl salicylate, bergamot oil, bitter orange oil, butylphenylmethylpropional, cardamom oil, cedrol, cinnamal, cinnamyl alcohol, citronellylmethyl crotonate, citrus oil, coumarin, diethyl succinate, d-limonene, ethyllinalool, eugenol, evernia furfuracea extract, evervnia prunastri extract, farnesol, guaiac wood oil, hexylcinnamal, hexyl salicylate, hydroxycitronellal, hydroxyisohexyl 3-cyclohexenecarboxaldehyde, lavender oil, lemon oil, linalyl acetate, mandarin oil, menthyl PCA, methylheptanone, nutmeg oil, rosemary oil, sweet orange oil, terpineol, tonka bean oil, triethyl citrate and/or vanillin.

11. Cosmetic preparation according to any one of the preceding claims, **characterized in that** the preparation comprises one or more diols selected from the group of the compounds 2-methyl-1,3-propanediol, pentane-1,2-diol, hexane-1,2-diol, heptane-1,2-diol, octane-1,2-diol, nonane-1,2-diol, decane-1,2-diol, ethylhexylglycerol.

12. Cosmetic preparation according to any one of the preceding claims, **characterized in that** preparation comprises one or more parabens (e.g. methylparaben, ethylparaben, propylparaben, butylparaben).

## Revendications

1. Préparation cosmétique sous la forme d'une crème de protection de la peau, d'une crème de jour ou de nuit, pour la protection solaire cosmétique ou produit de maquillage en cosmétique décorative, contenant :
a) du glutamate de stéaroyle sodique,
b) un polymère réticulé de vinylpyrrolidone et d'acide acrylique, à l'exclusion des formulations suivantes :
| | 1 | 2 | 3 | 4 |
|---|---|---|---|---|
| Stéarate de glycéryle | | | 2 | |
| Glutamate de stéaroyle sodique | 0,05 | 0,1 | 0,2 | 0,5 |
| Alcool stéarylique | | | 2 | |
| Copolymère de vinylpyrrolidone et d'acide acrylique | 2 | 1,6 | 0,5 | 1 |
| Carbomère | | 0,2 | | |
| Benzoate d'alkyle en C₁₂₋₁₅ | | 5 | 1 | 10 |
| Dicaprylate/dicaprate de butylène glycol | 2 | 8 | | 10 |
| Benzoate de phénéthyle | | | 2 | 5 |
| Octyldodécanol | | 2 | 4 | |
| Myristate de myristyle | | | | 1 |
| Triglycéride caprylique/caprique | | | 2 | |
| Néopentanoate isodécylique | 3 | | | |
| Cyclométhicone | 2 | | | |
| Diméthicone | 2 | | | |
| Glycérine | 10 | 2 | 5 | 8 |
| Bis-éthylhexyloxyphénol méthoxyphényltriazine | | 1 | | 2 |
| Butylméthoxydibenzoylméthane | 4 | 4 | | 3 |
| Polysilicone-15 | | 2 | 2 | |
| Méthoxycinnamate d'éthylhexyle | 3 | 3 | | |
| Salicylate d'octyle | | 3 | 2 | |
| Acide phénylbenzimidazole sulfonique | 1 | 0,5 | 1 | 2,5 |
| Acide phénylène-1,4-bis-(2-benzimidazyl)-3,3',5,5'tétrasulfonique | 1 | | 3 | 0,5 |
| Éthylhexyltriazine | 2 | | | 3 |
| Dioxyde de titane | 2 | | | |
| Octénylsuccinate d'amidon et d'aluminium | 2 | | | 2 |
| Octénylsuccinate d'amidon et de sodium | | | 3 | |
| Amidon de tapioca | 2 | | | |
| Alcool | | 4 | 4 | |
| Acide folique | | 0,03 | 0,02 | |
| Q10 | 0,05 | | | |
| Na₂H₂EDTA | 0,2 | 0,2 | 0,2 | 0,5 |
| Parfum, conservateur | q.s. | q.s. | q.s. | q.s. |
| Colorants, etc. | q.s. | q.s. | q.s. | q.s. |
| Hydroxyde de sodium | q.s. | q.s. | q.s. | q.s. |
| Eau | Jusqu'à 100,0 | Jusqu'à 100,0 | Jusqu'à 100,0 | Jusqu'à 100,0 |

2. Préparation cosmétique selon la revendication 1, **caractérisée en ce que** le composé de n° CAS : 27685-31-0 est utilisé en tant que polymère réticulé de vinylpyrrolidone et d'acide acrylique.

3. Préparation cosmétique selon l'une quelconque des revendications précédentes, **caractérisée en ce que** la préparation contient du polystéarate de sucrose.

4. Préparation cosmétique selon l'une quelconque des revendications précédentes, **caractérisée en ce que** la préparation contient du polyisobutène hydrogéné.

5. Préparation cosmétique selon l'une quelconque des revendications précédentes, **caractérisée en ce que** la préparation contient des électrolytes.

6. Préparation cosmétique selon l'une quelconque des revendications précédentes, **caractérisée en ce que** la préparation contient un ou plusieurs filtres UV choisis dans le groupe constitué par les sels de l'acide phénylène-1,4-bis-2-(benzimidazyl)-3,3'-5,5'-tétrasulfonique ; les sels de l'acide 2-phénylbenzimidazole-5-sulfonique ; le 1,4-di(2-oxo-10-sulfo-3-bornyliène-méthyl)-benzène et ses sels ; les sels de l'acide 4-(2-oxo-3-bornylidène-méthyl)benzène-sulfonique ; les sels de l'acide 2-méthyl-5-(2-oxo-3-bornylidène-méthyl)sulfonique ; les sels de l'acide téréphtalidène-dicamphre-sulfonique.

7. Préparation cosmétique selon l'une quelconque des revendications précédentes, **caractérisée en ce que** la préparation contient des filtres UV choisis dans le groupe constitué par les composés 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; salicylate d'éthylhexyle ; ester (2-éthylhexylique) de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester (2-éthylhexylique) de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque, 4-(tert-butyl)-4'-méthoxydibenzoylméthane ; salicylate d'homomenthyle ; 2-hydroxybenzoate de 2-éthylhexyle ; acrylate de 2-éthylhexyl-2-cyano-3,3-di-phényle ; benzalmalonate de diméthicodiéthyle ; acrylate de 2-éthylhexyl-2-cyano-3,3-diphényle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (n°CAS 288254-16-0) ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4-bis-{[4-(2-éthyl-hexyloxy)-2-hydroxy]-phényl}-6-(4-méthoxyphényl)-1,3,5-triazine ; 4-(tert.-butyl)-4'-méthoxydibenzoylméthane ; sels de N-(éthyloxysulfate-ester) de 4-dicyanométhylène-2,6-diméthyl-1,4-dihydropyridine, 2,4,6-tris-(biphényl)-1,3,5-triazine, 2,2'-méthylène-bis-(6-(2H-benzotriazol-2-yl)-4-(1,1,3,3-tétraméthylbutyl)-phénol), dioxyde de titane ; oxyde de zinc ; copolymère de polysiloxane ayant une distribution statistique selon la formule :

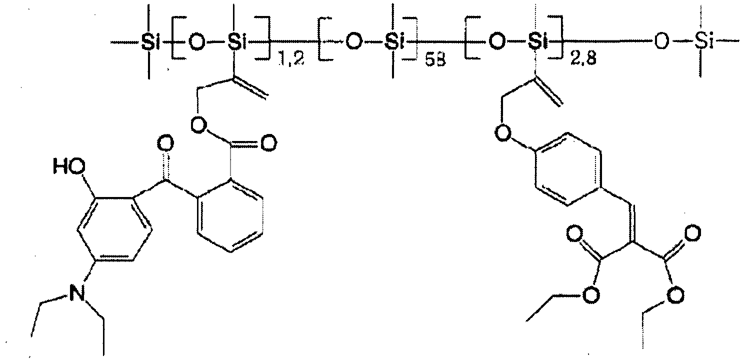

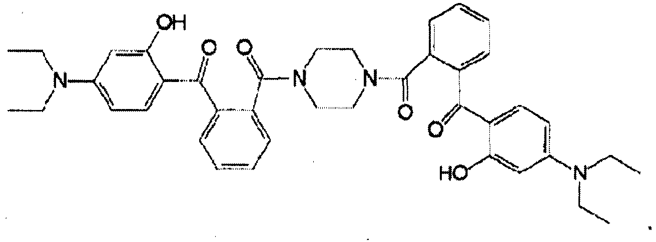

8. Préparation cosmétique selon l'une quelconque des revendications précédentes, **caractérisée en ce que** la préparation contient de l'amidon et/ou ses dérivés.

9. Préparation cosmétique selon l'une quelconque des revendications précédentes, **caractérisée en ce que** la préparation contient un ou plusieurs composés choisis dans le groupe constitué par les composés acide alpha-liponique, acide folique, phytoène, D-biotine, coenzyme Q10, alpha-glucosylrutine, carnitine, carnosine, isoflavonoïdes naturels et/ou synthétiques, flavonoïdes, créatine, créatinine, taurine, β-alanine, acétate de tocophéryle, dihydroxyacétone ; acide 8-hexadécène-1,16-dicarboxylique, glycérylglycose, (2-hydroxyéthyl)urée, vitamine E ou ses dérivés et/ou licochalcone A.

10. Préparation cosmétique selon l'une quelconque des revendications précédentes, **caractérisée en ce que** la préparation contient un ou plusieurs parfums choisis dans le groupe constitué par les composés limonène [5989-27-5], citral, linalool [78-70-6], alpha-isométhylionone [1335-46-2], géraniol [106-24-1], citronellol [106-22-9], [24851-98-7], [18479-58-8], [54464-57-2], [80-54-6], [1222-05,5], [32388-55-9], [105-95-3], [31906-04-4], [8008-57-9], [32210-23-4], [120-57-0], [115-95-7], [101-86-0], [140-11-4], [6259-76-3] et [127-51-5], 2-isobutyl-4-hydroxy-4-méthyltétrahydropyrane, acétate de 2-tert-pentylcyclohexyle, 3-méthyl-5-phényl-1-pentanol, 7-acétyl-1,1,3,4,4,6-hexaméthyltétraline, diester de l'acide adipique, alpha-amylcinnamaldéhyde, alpha-méthylionone, amyle C, cinnamate de butylphénylméthyl-propional, salicylate d'amyle, alcool amylcinnamylique, alcool anisique, benzoïne, alcool benzylique, benzoate de benzyle, cinnamate de benzyle, salicylate de benzyle, huile de bergamote, huile d'orange amère, butylphénylméthylpropional, huile de cardamome, cédrol, cinnamal, alcool cinnamylique, crotonate de citronellylméthyle, huile de citron, coumarine, succinate de diéthyle, d-limonène, éthyllinalool, eugénol, extrait d'Evernia Furfuracea, extrait d'Evernia Prunastri, farnésol, huile de guaïc, hexylcinnamal, salicylate d'hexyle, hydroxycitronellal, hydroxyisohexyl-3-cyclohexène-carboxaldéhyde, huile de lavande, huile de citron, acétate de linalyle, huile de mandarine, menthyl PCA, méthylhepténone, huile de noix de muscade, huile de romarin, huile d'orange douce, terpinéol, huile de fève de tonka, citrate de triéthyle et/ou vanilline.

11. Préparation cosmétique selon l'une quelconque des revendications précédentes, **caractérisée en ce que** la préparation contient un ou plusieurs diols choisis dans le groupe constitué par les composés 2-méthyl-1,3-propanediol, pentane-1,2-diol, hexane-1,2-diol, heptane-1,2-diol, octane-1,2-diol, nonane-1,2-diol, décane-1,2-diol, éthylhexylglycérine.

12. Préparation cosmétique selon l'une quelconque des revendications précédentes, **caractérisée en ce que** la préparation contient un ou plusieurs parabènes (p. ex. méthyl-, éthyl-, propyl-, butylparabène).
